# EUROPEAN PATENT APPLICATION

(11) **EP 1 806 114 A2**
(43) Date of publication of application: **11.07.2007**
(21) Application number: 07250008.5
(22) Date of filing: 03.01.2007
(51) Int. Cl.: A61F 2/84

(54) **Handle system for deploying a prosthetic implant**

(30) Priority: 04.01.2006 US 324902
(71) Applicant: Cordis Corporation, Miami Lakes, FL 33014 (US)
(72) Inventor: Cioanta, Iulian, Weston FL 33327 (US); Rincon, Cesar, Coral Springs FL 33065 (US); Wilkins, Brian W., Miami Beach FL 33139 (US); Winkler, Rance A., Miramar FL 33029 (US)
(74) Representative: Belcher, Simon James

(57) **Abstract**

A handle system of a delivery catheter for the deployment of prosthetic implants includes a first stationary portion, at least one guide rail secured to the first stationary portion and extending generally longitudinally with the handle system, and a generally cylindrical second rotating portion rotatably connected to the first stationary portion. A sheath mount is secured to an outer sheath of the delivery catheter, and includes one or more bearing surfaces to engage with at least one of the one or more guide rails. Rotation of the second rotating portion longitudinally displaces the sheath mount by interaction with the sheath mount. Interaction may be by internal thread of the rotating portion and external thread of the sheath mount, or alternately by rotating one or more drive screws by turning the rotating portion, the drive screws interacting with through holes in the sheath mount configured to receive and engage the drive screws.

## Description

The invention relates to the field of medical devices, and more particularly to a handle system of a catheter for the deployment of a prosthetic implant.

Vascular disease is a leading cause of premature mortality in developed nations, often presenting as a vascular aneurysm. A vascular aneurysm is a localized dilation of a vessel wall, due to thinning or weakness of the wall structure, or separation between layers of the vessel wall. If untreated, the aneurysm may burst and haemorrhage uncontrollably. Aneurysms are particularly dangerous and prevalent in the aorta, because the aorta supplies blood to all other areas of the body, and because the aorta is subject to particularly high pressures and stresses accordingly. Rupture of an aortic aneurysm is the 15^{th} leading cause of death in the United States, afflicting 5% of older men.

Aortic aneurysms are described by their position. They are either thoracic, generally between the aortic arch and the junction of the left and right renal arteries, or abdominal, between the junction of the renal arteries and the branch of the iliac arteries.

It is known to treat aortic aneurysms surgically where blood pressure control medication is unsuccessful at arresting growth of the aneurysm. Surgery often involves the insertion of a vascular stent graft to exclude the aneurysm and carry blood past the dilated portion of the vessel, relieving the pressure on the aneurysm. Designing a viable stent graft for the treatment of abdominal aortic aneurysm (AAA) is particularly challenging, in part because the graft must branch to follow the shape of the abdominal aorta to carry blood into the separate iliac arteries without obstruction.

Moreover, it would be advantageous to design a stent graft that is collapsible to facilitate percutaneous insertion by minimally invasive surgical techniques. Additionally, percutaneous insertion requires the design and development of a delivery system that can effectively position and deploy the vascular stent.

Towards this end, modular stent grafts have been developed in which a bifurcate first portion is located in the abdominal aorta, while additional portions extend beyond the first portion, for example into the iliac vessels. However, deployment in such vessels has proven challenging. Moreover, part of that challenge has been the design of a handle system for a delivery catheter, operable by a surgeon at the proximal end of the delivery catheter which can deploy a stent graft implant remotely at the distal end of the delivery catheter.

Accordingly, in one aspect, the present invention provides a handle system of a delivery catheter for the deployment of prosthetic implants. The handle system includes a first stationary portion, at least one guide rail secured to the first stationary portion and extending generally longitudinally with the handle system, and a generally cylindrical second rotating portion rotatably connected to the first stationary portion, the second rotating portion having a threaded internal surface. A sheath mount is secured to an outer sheath of the delivery catheter, an includes one or more bearing surfaces to engage with at least one of the one or more guide rails, and a generally cylindrical outer surface with a configuration to engage the internal threaded surface of the second rotating portion. Rotation of the second rotating portion longitudinally displaces the sheath mount by interaction of the threaded internal surface with the configuration of the generally cylindrical outer surface.

The threaded internal surface can be a helical male thread, preferably a four-start thread, and the configuration of the generally cylindrical outer surface comprises a helical channel, preferably a four start channel. Alternately , the threaded internal surface comprises a thread channel, and the configuration of the generally cylindrical outer surface comprises one or more protrusions sized to engage the thread channel. In that configuration, thread channel can vary in pitch along the longitudinal axis of the second rotating portion, with greater pitch at a proximal section.

The guide rail can include ratchet teeth along its length, and the sheath mount a ratchet arm to engage the ratchet teeth and permit movement of the sheath mount in a first direction, but inhibit movement in the opposite direction. The guide rail can further have a dead zone, preferably at a distal end of the guide rail, where no ratchet teeth are present.

The sheath mount may include a locking button captured in a radial recess, biassed radially outward by a biasing means, such as compression spring, with the second rotating portion having one ore more through holes sized to admit at least a portion of the locking button. First stationary portion can include a corresponding release button longitudinally aligned with one or all through holes, to depress the locking button radially inward of the through hole. The release button may be captured to the first stationary portion, and include a lock to retain the release button in the depressed position.

A strain relief, formed for example from PTFE or polyethylene, joined to the first stationary portion at a distal tip of the handle system can alleviate kinking in the delivery catheter. For increased flexibility, the strain relief can have longitudinal ribs with longitudinal spaces between them. The longitudinal ribs define a generally conical outer surface, and support an inner cylinder, and may penetrate the inner cylinder and/or extend to the distal tip of the strain relief. Alternately, plural rows of openings extending a circumferential direction, preferably staggered in longitudinal and circumferential directions, can be provided

The handle system preferably includes one or more circumferential ribs extending at least partially around the first stationary portion, and/or one or more longitudinal ribs extending at least partially along the second rotating portion.

The handle system is may be part of a delivery catheter extending from the distal tip of the stationary portion, the delivery catheter having an inner core and an outer sheath which are longitudinally displaceable relative to one another. The delivery catheter can be preloaded with one or more prosthetic implants at a distal tip thereof.

The handle system can have a first stationary portion and at least one guide rail secured to the first stationary portion and extending generally longitudinally with the handle system. A second rotating portion is rotably connected to the first stationary portion, the rotating portion having a central opening with internal gear teeth. One or more drive screws extends generally longitudinally with the handle system. Each drive screw has a spur gear at a proximal end thereof, the spur gears communicating with the internal gear teeth of the central opening to rotate together with the second rotating portion. A sheath mount is secured to an outer sheath of the delivery catheter, and includes one or more bearing surfaces to engage with at least one of the one or more guide rails. Sheath mount also has one or more through holes with a configuration to engage one or more drive screws, in which rotation of the second rotating portion longitudinally displaces the sheath mount by interaction of the drive screws with the configuration of the one or more through holes in the sheath mount.

The drive screws can be provided as two sets, preferably with an equal number of drive screws in each set. The first set of the drive screws can engage the internal gear teeth via an idler gear to reverse the direction of rotation, with the first set of the drive screws threaded in the opposite direction from the second set.

Embodiments of the invention will now be described by way of example with reference to the accompanying drawings, in which:
Fig. 1 is a perspective view of the handle system of the invention;
Fig. 2 is a sectional view of a portion of the handle system of Fig. 1;
Fig. 3 is a sectional view of a medial portion of the handle system of Fig. 1;
Fig. 4 is an exploded assembly view of a sheath mount of the invention;
Fig. 5 is a longitudinal cross section through another embodiment of the sheath mount of the invention;
Fig. 6 is an assembly view of the sheath mount shown in Fig. 5;
Fig. 7 is a longitudinal cross section of the handle system;
Fig. 8 is a longitudinal cross section through a distal portion of the handle system;
Fig. 9 is a longitudinal section of a proximal portion of the handle system;
Fig. 10 is a longitudinal section of a further embodiment of the delivery handle;
Fig. 11 is a longitudinal cross section of a lock and release button in the locked configuration;
Fig. 12 is a longitudinal cross section of a lock and release button in the unlocked configuration;
Figs. 13A-13F show various embodiments of strain relief arrangements in a handle system according to the invention;
Fig. 14 is an internal assembly view of a delivery catheter handle system;
Fig. 15 is a graph showing how torque varies with sheath mount displacement for two handle systems according to the invention;
Fig. 16 is a partial assembly view of another handle system according to the invention;
Fig. 17 is a stationary portion of the delivery handle associated with the embodiment of Fig. 16;
Fig. 18 is a proximal end of the handle system according to the embodiment of Fig. 16; and
Fig. 19 is a variety of differing configurations of a handle system 10 according to the present invention.

In a delivery system for a catheterized implant device, the implant, for example a stent or stent graft, is radially compressed onto an inner shaft or central core of the catheter. The implant and the inner shaft are then covered by an outer sheath, which restrains the implant during insertion into the body. Once delivered to the deployment site, the outer sheath is retracted, releasing the implant to expand to its deployed diameter. The location and deployment of the implant is controlled remotely at the proximal handle of the delivery catheter, minimizing trauma to the patient.

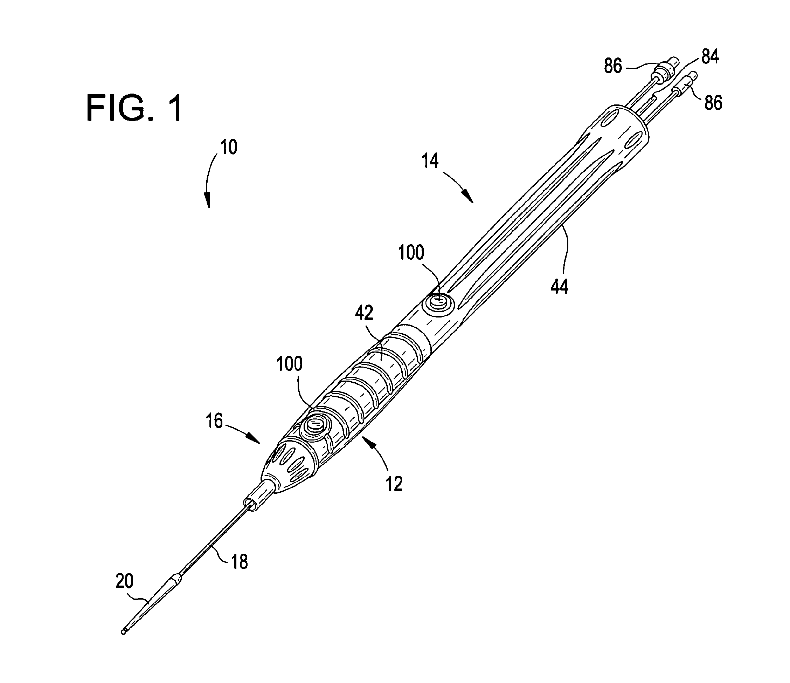

Referring to the drawings, Fig. 1 shows a handle system 10 which has a stationary portion 12 rotatably connected to a rotating portion 14. Stationary portion 12 is considered stationary with respect to the handle system 10, and to the larger delivery catheter system of which the handle system 10 is a part. Stationary portion 12 is moveable by the surgeon as part of manipulating the handle system 10 and associated delivery catheter. Strain relief 16 extends distally from the stationary portion 12, and provides strain relief for the catheter 18. Catheter 18 extends distally from the handle system 10 to a distal tip 20. Fig. 1 shows catheter 18 much shorter than it would be in most applications, solely for ease of illustration. Catheter 18 can be, and in most instances is, considerably longer.

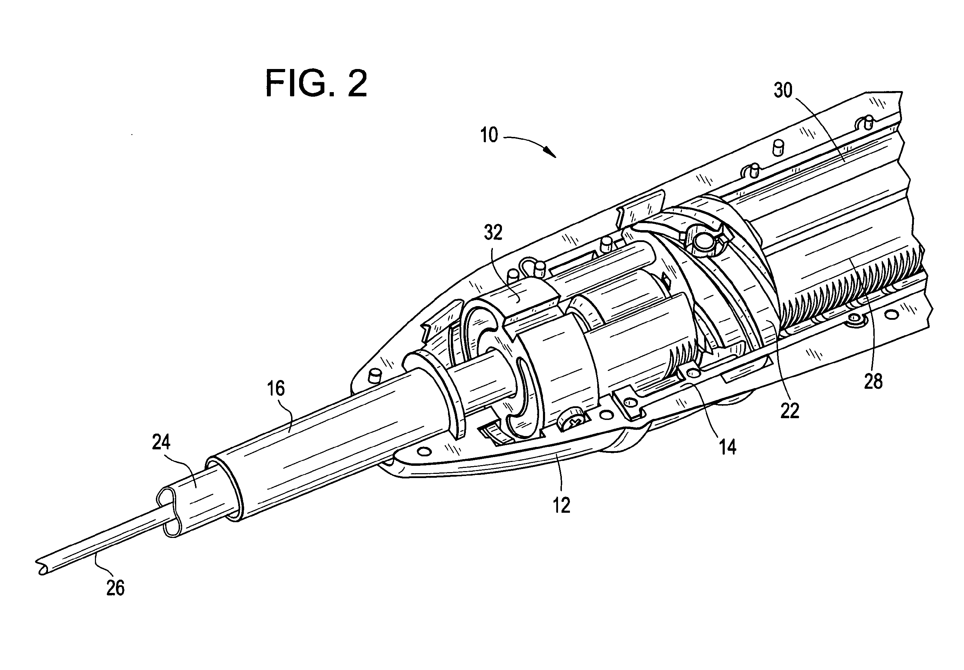

Fig. 2 is sectional view of the handle system 10, and more particularly stationary portion 12. Catheter 18 can be seen extending from strain relief 16. Shown in cutaway view is the outer sheath 24 and inner shaft 26 of catheter 18. Two longitudinal rails, 28, 30, extend internally through the handle system 10, and are secured to the stationary portion 12, in this case via rail lock bracket 32. Rotating portion 14 can extend into stationary portion 12, in this case almost to strain relief 16. As will be described, infra, the length of the rotating portion 14 limits the travel of sheath mount 22, and consequently, the amount by which the outer sheath 24 will be retracted. Moreover, the overall length of the handle system 10 can be advantageously limited by minimizing the extension of the stationary portion 12 beyond the rotating portion 14.

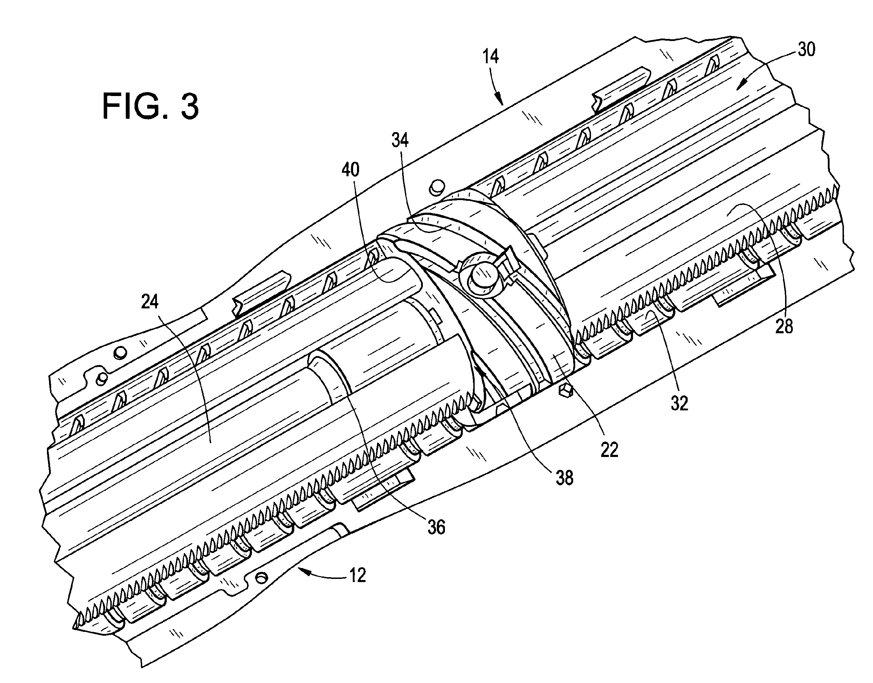

Referring now to Fig. 3, illustrated is a sectional view of the handle system 10, particularly a medial portion thereof. The proximal end of stationary portion 12 can be seen, as well as rotating portion 14. Rotating portion 14 has an internal helical thread 32, which mates with an external thread 34 around the exterior of sheath mount 22. Preferably, the internal thread 32 comprises 1.00 in diameter, 4-start, having a .945 inch pitch, with external thread 34 sized to match accordingly. The 4-start thread is selected to more evenly distribute the forces around the circumference of the sheath mount 22 as compared with a helical thread having fewer starts. Outer sheath 24 is secured to sheath mount 22 at a central mounting nipple 36. Sheath mount 22 rides along rails 28, 30, and has rail bearings 38, 40 for that purpose. Rails 28, 30, and corresponding bearings 38, 40, are preferably kidney-shaped in cross section, to provide improved resistance to torque while maintaining increased central clearance for the passage of the catheter 18 through the centre of the handle system 10.

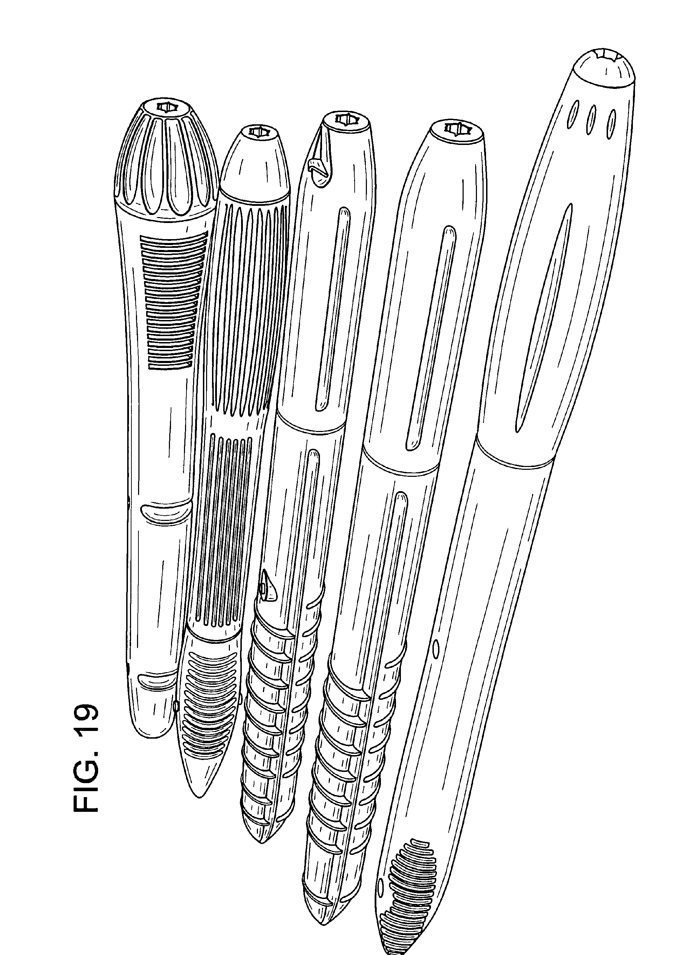

The handle system is operated to deploy an implant by rotating the rotating portion 14, while holding the stationary portion 12 fixed. The internal thread 32 drives the external thread 34 of sheath mount 22 in a proximal direction of the handle system 10. Accordingly, outer sheath 24, being secured to sheath mount 22, is retracted proximally to expose the implant at a distal end of the delivery catheter, and allowing it to deploy. Accordingly, to enhance this functionality, the stationary portion 12 is preferably provided with circumferential ribs 42 (See Fig. 1). Longitudinal ribs assist the surgeon in maintaining the position of the handle system 10 against the axial force imparted by the retraction of the outer sheath 24. Additionally, rotating portion 14 is preferably provided with longitudinal ribs 44, to improve tactile control by the surgeon. This is particularly useful considering the surgeon will be wearing barrier gloves during the procedure. Fig. 19 illustrates various differing embodiments of a handle system 10 according to the present invention, showing a variety of configurations possible within the scope of the present invention.

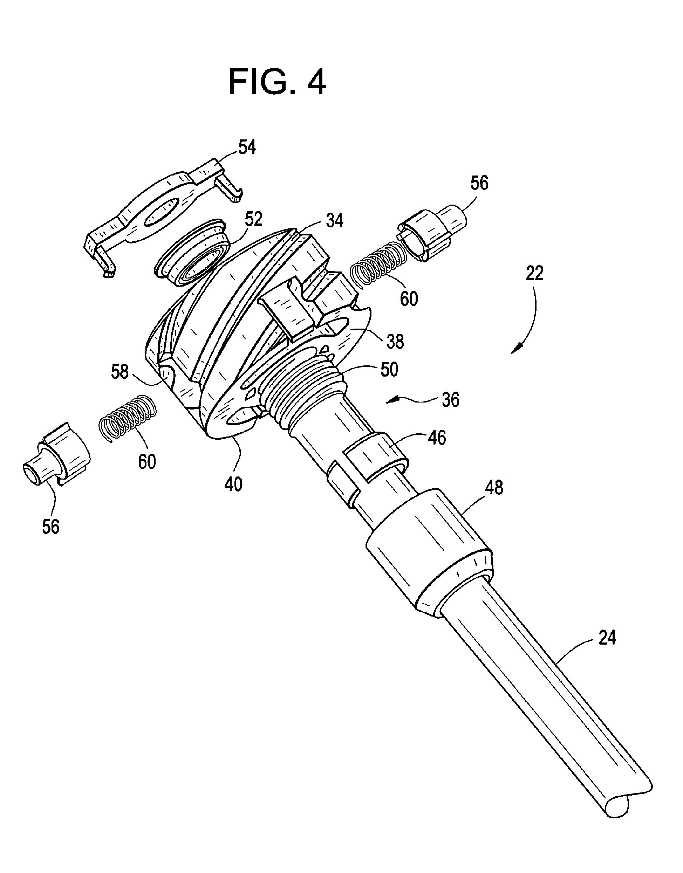

Fig. 4 is an exploded assembly view of sheath mount 22. Outer sheath 24 is advanced over mounting nipple 36. A collar 46 surrounds the outer sheath 24 and mounting nipple 36 along the length of the mounting nipple 36. An internally threaded ferrule 48 is advanced over the collar 46, outer sheath 24 and nipple 36 and threaded to the sheath mount 22 at external thread 50. As the threaded ferrule 48 is tightened, it compresses collar 46, and forms a fluid-tight seal between the outer sheath 24 and the sheath mount 22. Alternate means of forming the fluid tight seal between the outer sheath 24 and the sheath mount 22 are contemplated, and will be illustrated with respect to the additional figures.

Referring still to Fig. 4, the inner shaft seal 52 forms a fluid tight seal against the exterior of the inner shaft 26 of the catheter, which passes through the centre of the sheath mount 22. Inner shaft seal 52 is preferably a flexible material, and is secured in place to the sheath mount by seal retainer 54, which preferably snap-fits to sheath mount 22. One or more lock buttons 56, are secured in recesses 58, and biassed radially outward by biassing means, for example springs 60. Where multiple lock buttons 56 are provided they are preferably evenly spaced around the circumference of the sheath mount 22. In this case, two lock buttons 56 are diametrically opposed. Lock buttons 56 extend through recesses in the rotating portion 14 and/or stationary portion 12 to lock the sheath mount against movement, as will be illustrated further, infra.

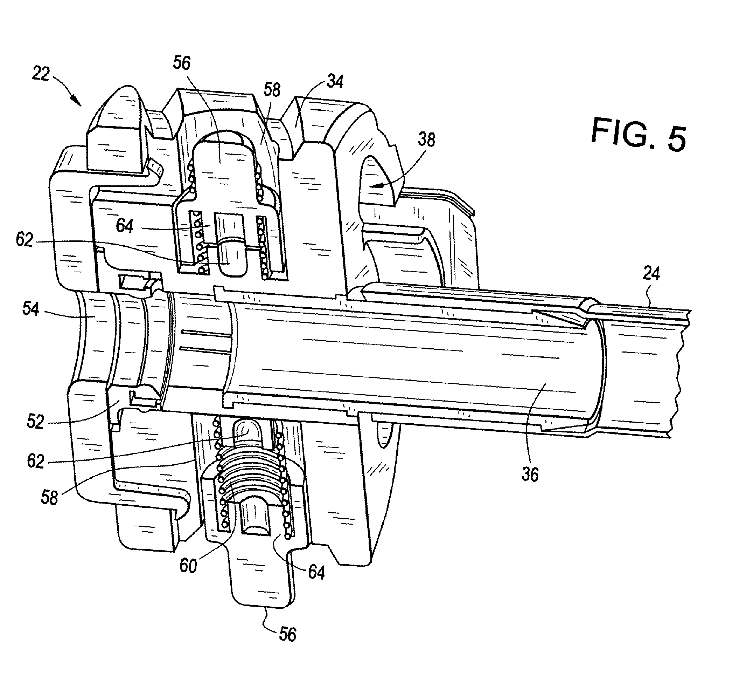

Fig. 5 is a longitudinal cross section through an alternate embodiment of sheath mount 22. In this embodiment, mounting nipple 36 is a 303/304 stainless steel insert moulded with a centre barb. Outer sheath 24 has an integrally moulded plastic collar that is secured over the mounting nipple 36 and snap fit onto the sheath mount 22. This gives certain manufacturing advantages over the previously described mounting arrangement. An externally threaded post 62 at the bottom of each recess 58 secures the spring 60. Lock button 56 has an externally threaded post 64 which similarly is secured to spring 60. In this way, the lock button is captured in recess 58. However, this is not the exclusive methods of capturing lock buttons 56 according to the present invention, and alternate arrangements may be apparent to those skilled in the art in light of this disclosure.

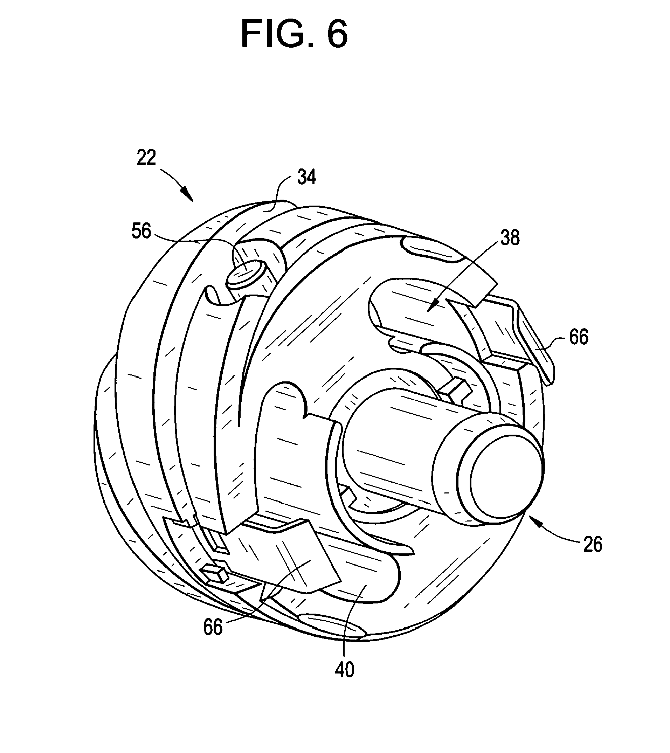

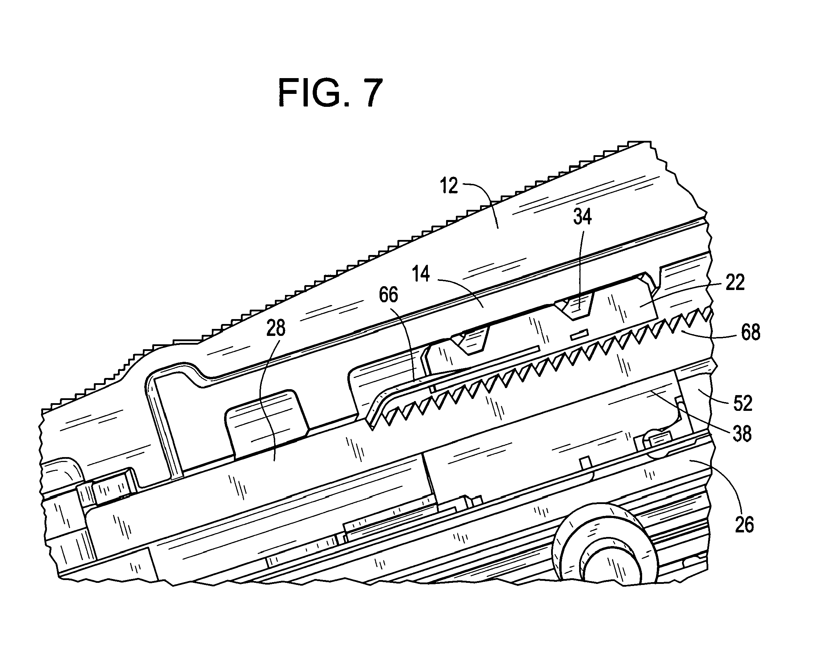

Fig. 6 is an assembly view of sheath mount 22 according to the alternate embodiment of Fig. 5. As illustrated in Fig. 6, sheath mount 22 includes ratchet arms 66 on either side of bearings 38, 40. Referring now to Fig. 7, a longitudinal cross section of the handle system 10, ratchet arm 66 mates with ratchet teeth 68 provided on longitudinal rail 28. Similar teeth may be provided on opposing rail 30. Ratchet arm 66 together with ratchet teeth 68 prevent distal movement of the sheath mount 22 once retracted. They also give the surgeon an audible click indicating a predetermined length of retraction of the sheath mount 22 and outer sheath 24. Preferably, the ratchet teeth are provided at 1 mm intervals, though almost any interval may be adopted.

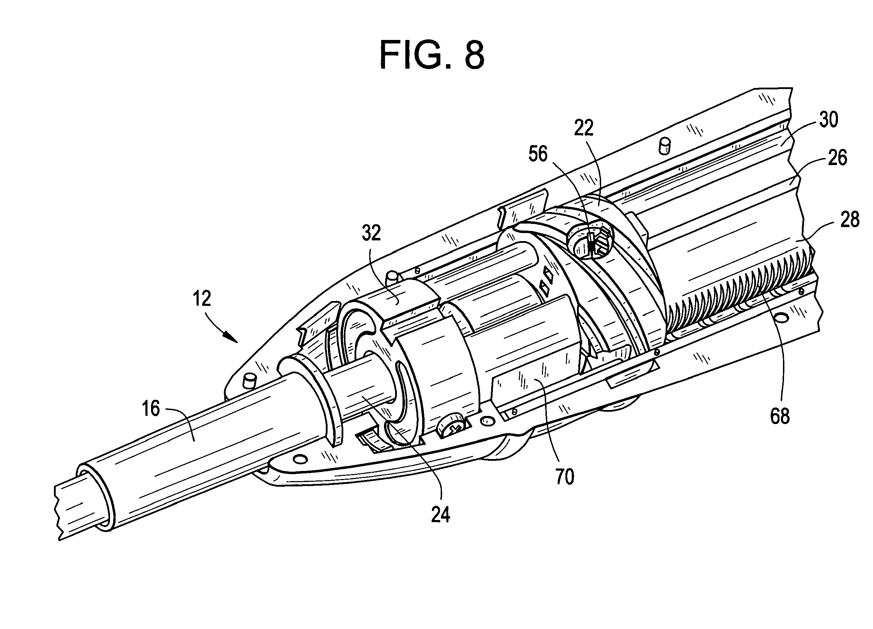

As shown in Fig. 8, rails 28, 30 preferably have a dead zone 70 at the distal ends thereof where no teeth inhibit the movement of sheath mount 22, in addition to ratchet teeth 68. It is often the case that the surgeon will wish to recapture the implant after initiating deployment, for example to correct or improve its location. This is most common in the early stages of deployment. Therefore, it is desirable to be able to advance the outer sheath 24 after at least some retraction, in order to recapture the implant. Therefore, a dead zone 70 is provided where no ratchet teeth 68 inhibit the motion of the sheath mount 22.

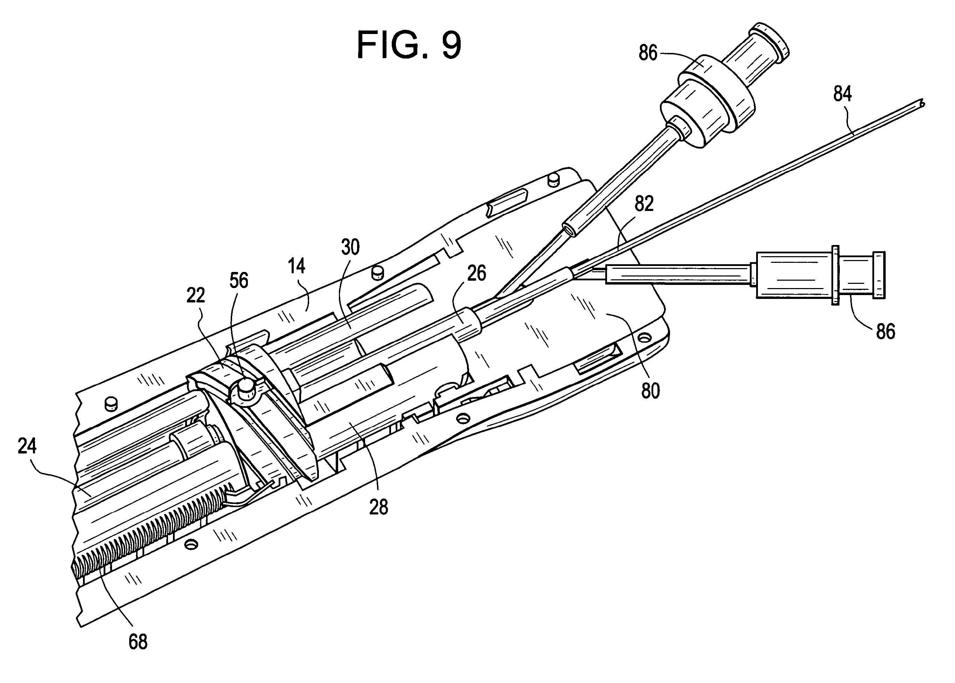

Fig. 9 is a longitudinal section of the handle system 10, and more particularly a proximal end thereof. Longitudinal rails 28, 30 extend to a proximal manifold 80. Manifold 80 seals the inner shaft 26. An axial lumen 82 permits a guide wire 84 to pass through the manifold 80 and into the catheter. Optionally, luer connectors 86 permit introduction of fluids or agents into the manifold and the catheter by injection with a syringe.

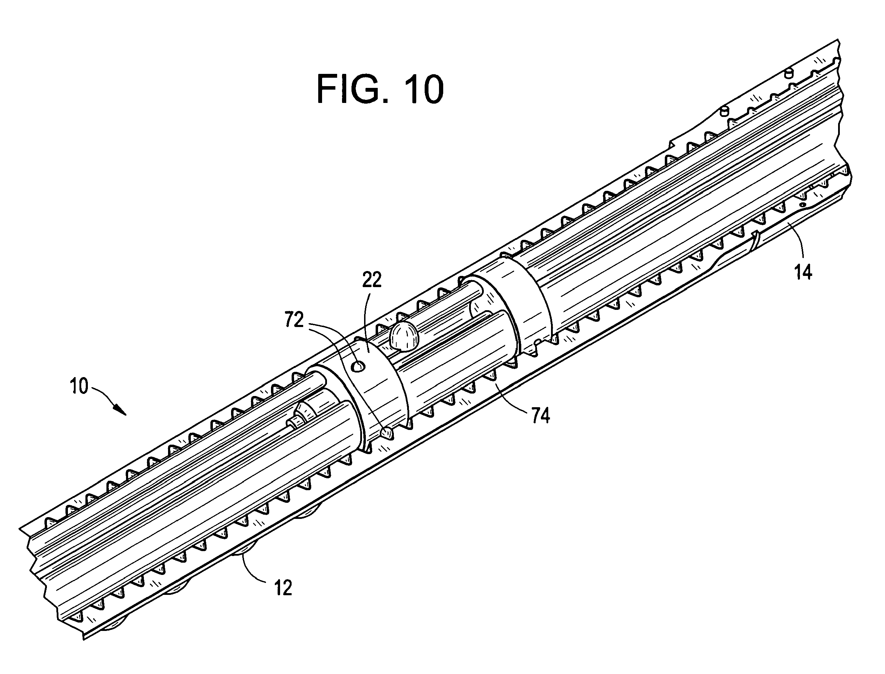

It is often desired to retract the outer sheath 24 more slowly in the initial stages of deployment, to ensure accurate placement of the implant. However, once the distal end of the implant is properly deployed, there is no reason to delay the full retraction of the outer sheath 24. Therefore, referring to Fig. 10, illustrated in longitudinal section is an alternate embodiment of the delivery handle 10.

Fig. 10 shows a sheath mount 22 which has one or more, preferably four, protrusions 72 extending radially outward from the sheath mount 22. Protrusions 72 are preferably spherical in shape, and may be a sphere at least set into a recess of the sheath mount 72 provided for that purpose. Moreover, protrusions 72 are preferably evenly distributed around the circumference of the sheath mount 22. In the case of four protrusions 72, they are placed at 90 degrees from the adjacent protrusion.

As can be seen in Fig. 10, rotating portion 14 has one or more recessed groves 74 for receiving one or more of protrusions 72. Preferably, there is at least one recessed grove 74 for receiving each protrusion 72. The recessed groves 74 preferably define a helical path. Therefore, four recessed groves 74 can define a four-start helical path, in a similar manner to thread 32 of the previously described embodiment. Additionally, the recessed groves 74 can define an arbitrary pitch that varies over the length of the rotating portion 14. Preferably, as described, supra, the recessed groves 74 define a fine pitch at the distal portion. Accordingly, the sheath mount 22 and attached sheath 24 will retract more slowly for a given rate of rotation of the rotating portion 14. After some predetermined length of rotating portion 14, the pitch of recessed grooves 74 may increase, to provide faster retraction. The described arrangement will be seen as merely exemplary, and able to accommodate nearly any variable pitch arrangement.

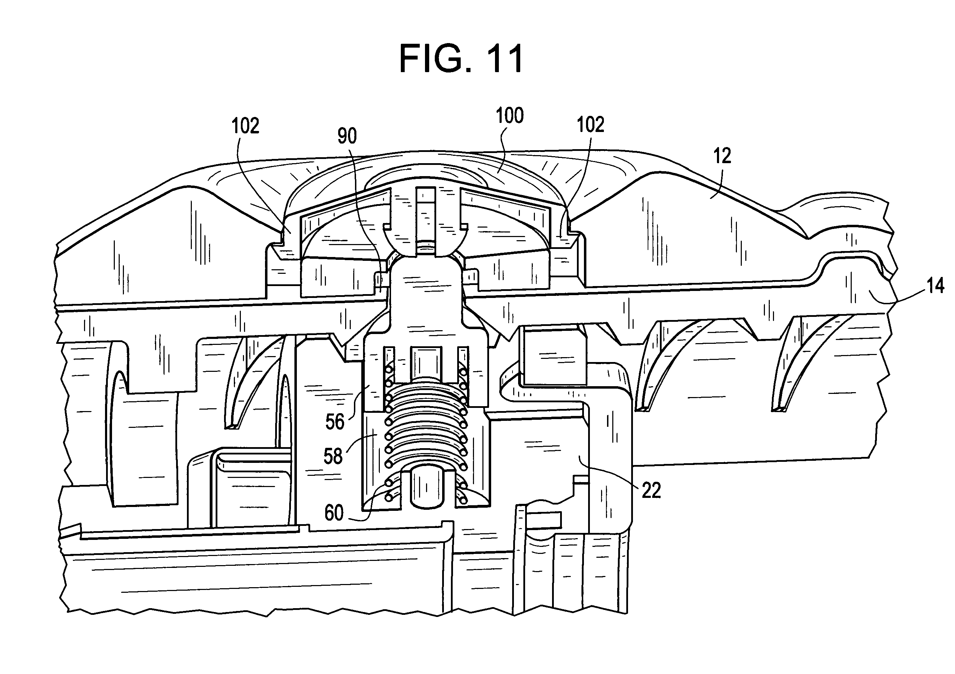

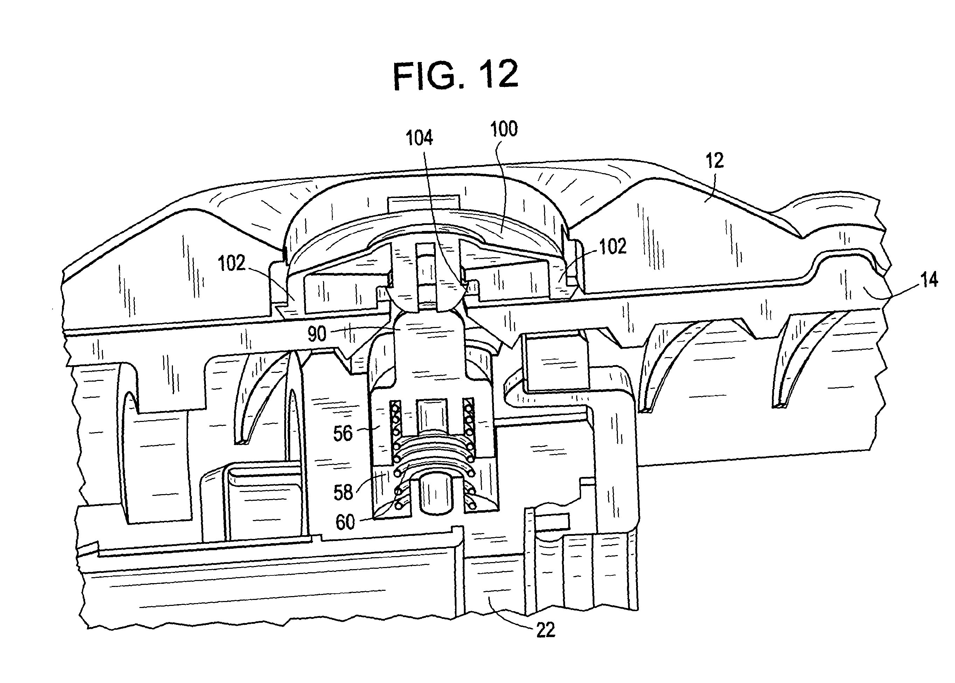

Figs. 11 and 12 are longitudinal cross sections through a lock and release button according to a preferred embodiment of the present invention in the locked and unlocked configurations respectively. Where sheath mount 22 is provided with lock buttons 56, associated recess 58, and biassing springs 60, the rotating portion 14 of the handle system 10 includes a through hole 90 sized to pass at least a portion of lock button 56. Stationary portion 12 has a corresponding release button 100 located in a longitudinally aligned position with through hole 90. When properly aligned, lock button 56, under bias of spring 60, extends outward via through hole 90 to lock the sheath mount 22 against any axial movement, and thereby to lock the rotating portion 14 against any rotation.

As shown in Fig. 12, the release button 100 is preferably captured to stationary portion 12, for example by snap-locks 102. Release button 100 has at least enough freedom of motion to depress lock button 56 inside of rotating portion 14. On doing so, the lock button 56 no longer inhibits any motion of sheath mount 22 or rotating portion 14. Release button 100 preferably has depression locks 104 to capture release button 100 in the lower unlocked position once depressed. Therefore, the lock button would be prevented from re-engaging through hold 90.

Where the delivery catheter including handle system 10 is pre-loaded with an implant, the handle system 10 is preferably locked by lock button 56 and through hole 90 with the outer sheath 24 and sheath mount 22 in a distal-most advanced position. Accordingly, any premature retraction of outer sheath 24 and sheath mount 22 is prevented until a surgeon depresses release button 100. Further, where the delivery catheter is preloaded with plural implants, or a multi-part implant, more than one through hole 90 and release button 100 pairs can be provided along the length of the handle system 10. Preferably, upon reaching the predetermined retraction distance to deploy a first implant or first part of an implant, the handle system can re-lock at the intermediate position. This positively indicates the position of the sheath mount 22 to the surgeon. The delivery catheter can then be repositioned to deploy a second implant or second stage. Once repositioned, the surgeon can begin the second deployment by pressing a second intermediate release button 100.

Figs. 13A to 13F show various strain relief arrangements 16. For example, in a first embodiment Fig. 13A, strain relief 16a is conically shaped and has a generally solid wall of constant thickness. In the exemplary embodiments, the strain relief 16a material comprises polytetrafluoroethylene (PTFE), though other plastics may be substituted as well, including without limitation polyethylene or variations thereof. Embodiment 16a is considered less preferred because it is too stiff to provide enough flexure to avoid kinking of the delivery catheter when the handle system 10 is turned transversely at angles approaching 90° or more relative to the delivery catheter. Therefore, strain reliefs 16b-16f are offered as alternate embodiments having more preferred performance characteristics.

In each of embodiments 16b-16f, it will be seen that some material is removed relative to strain relief 16a. In the embodiment of Fig. 13B, strain relief 16b has longitudinal ribs 110 supporting an inner cylinder 112. Spaces 116 separate ribs 110. Ribs 110 increase in thickness away from the tip 114 to form a generally conical shape. In the embodiment of Fig. 13C, strain relief 16c, spaces 116 penetrate inner cylinder 112, which is defined generally by ribs 110.

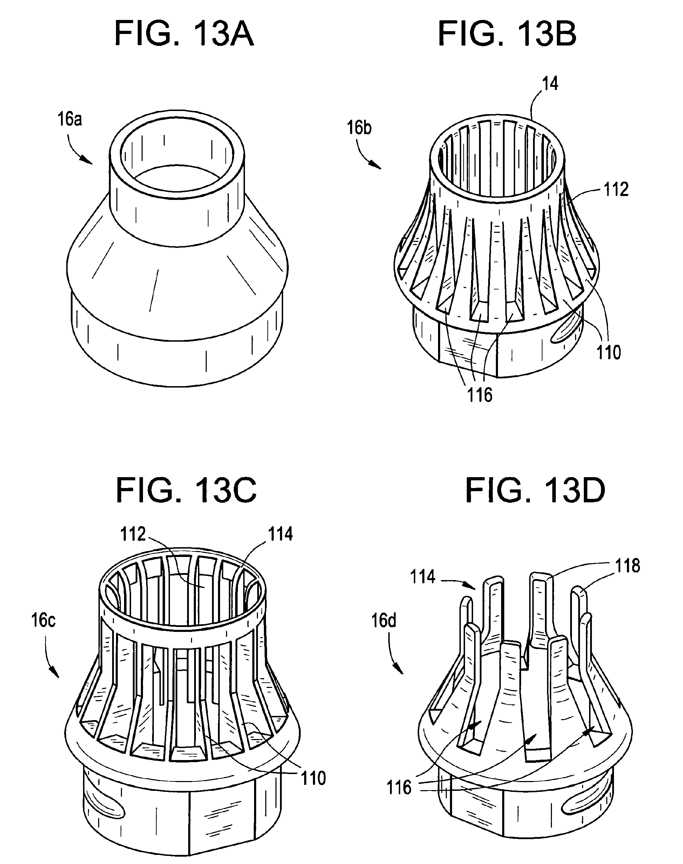

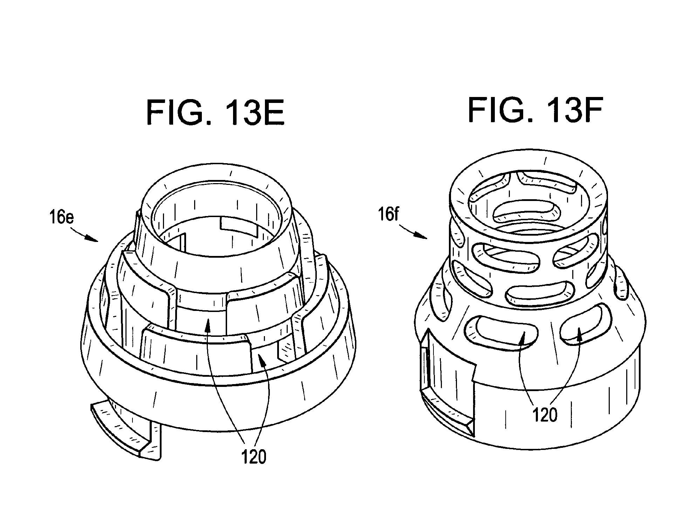

In the embodiment of Fig. 13D, strain relief 16d is characterized by fingers 118 8 extending longitudinally, separated by spaced 116. Fingers 118 have no connection to one another at the distal tip 114. In the embodiment of Fig. 13E, strain relief 16e features rows of circumferential openings 120 spaced around the embodiment 16e. Openings 120 are staggered from adjacent openings 120 both longitudinally and circumferentially. Similarly, in the embodiment of Fig. 13F, strain relief 16f features circumferentially and longitudinally staggered openings 120.

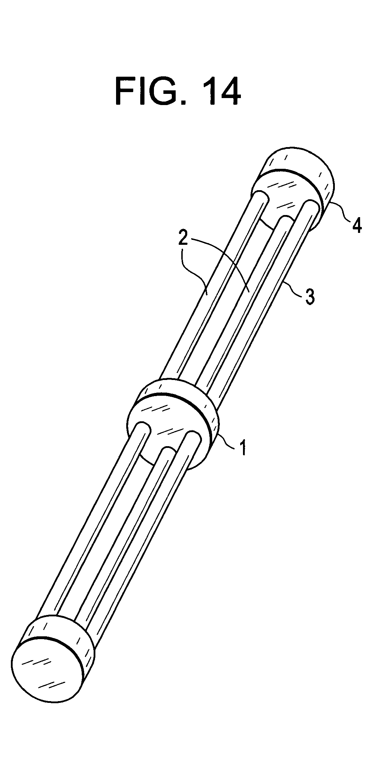

Referring to Fig. 14, illustrated is a internal assembly view of a less preferred embodiment of a delivery catheter handle system rendered as a finite element stress analysis representation. In the less preferred embodiment, the sheath mount 1 is guided and displaced by three rails spaced around the centre of the sheath mount 1 and passing through it. Two are solely guide rails 2, the third is a lead screw 3, which is turned to axially displace the sheath mount 1. The handle system 10 according to the preferred embodiments is a marked improvement for several reasons. First, the previous design was subject to a great stress concentrations at 4, the proximal end of shaft 3. This problem is ameliorated in part by distributing the driving force around the circumference of the sheath mount 22.

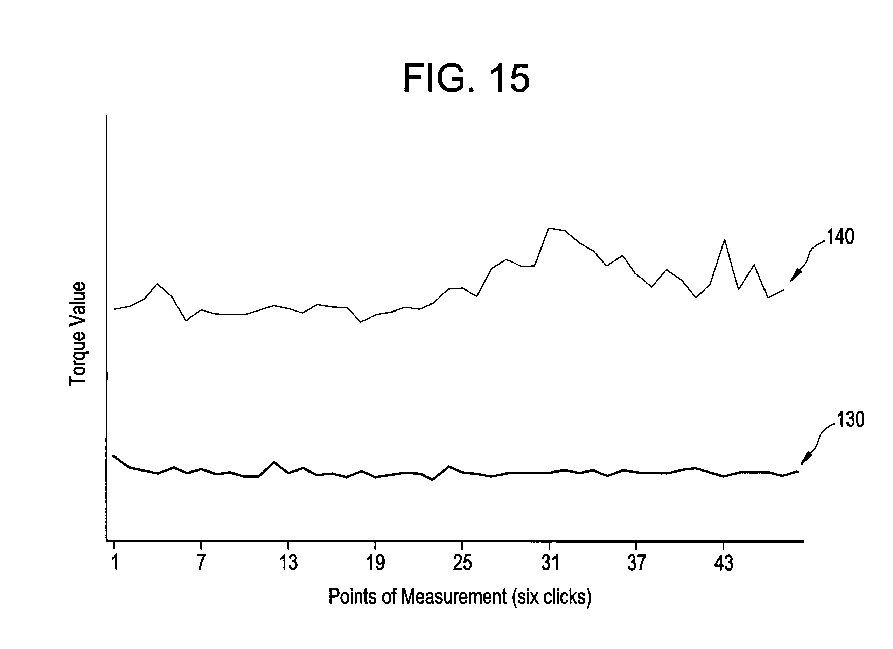

Second, and referring to Fig. 15, torque required (Y-axis) to displace the sheath mount 22 is almost 70% less in the preferred embodiment described in Figs. 1-12, line 130, than the less preferred embodiment of Fig. 14, line 140. Less torque results in less fatigue to the surgeon, and a more accurate deployment of the implant. Additionally as seen in Fig. 15, the applied torque remains relatively constant over the displacement of the sheath mount 22. This compares to the previous embodiment, where torque increases and fluctuates as the outer sheath is displaced (X-axis). This also improves the accuracy and ease of implant deployment.

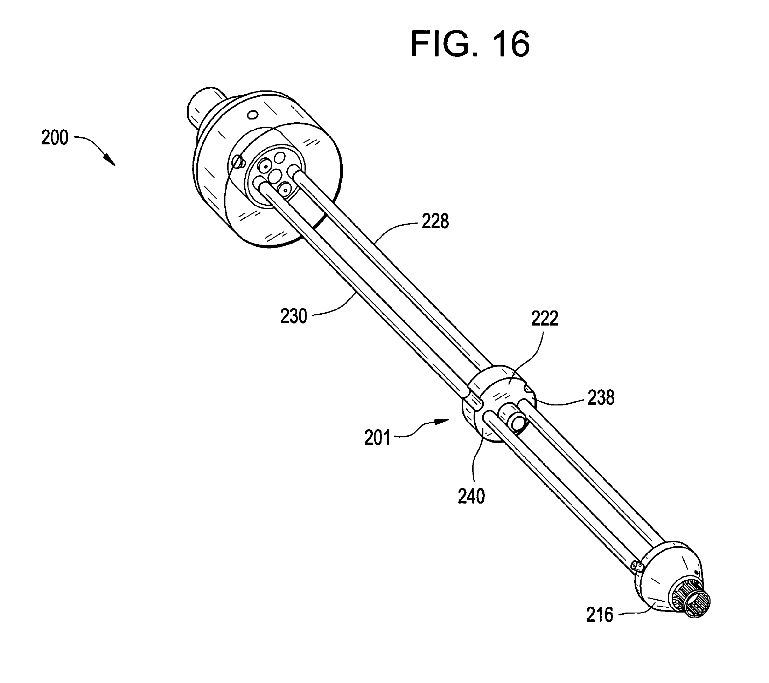

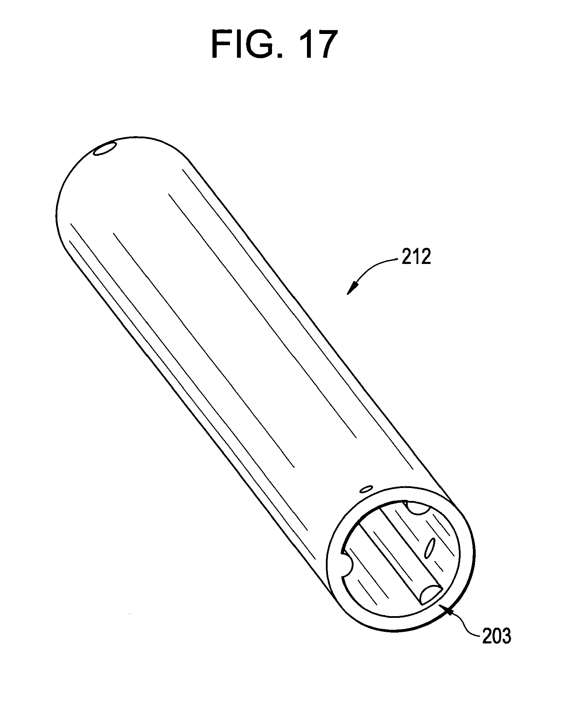

Fig. 16 is a partial assembly view of a handle system 200 according to yet another embodiment of the present invention. Construction and operation of the second embodiment will generally be appreciated from the foregoing description of the first embodiment, and therefore only certain salient distinguishing features will be described in detail. Sheath mount 222 is guided longitudinally along rails 228, 230 by bearing surfaces 238, 240. Rails 228, 230 extend from a strain relief 216 at the distal tip of the handle system 200. Preferably, one or more longitudinal recesses 201 are distributed around the circumference of sheath mount 222, in the exemplary embodiment there are three. Referring to Fig. 17, recesses 201 receive protrusions 203 of a stationary portion 212 of the handle system 200. Stationary portion 212 generally surrounds and encloses the assembly illustrated in Fig. 16. Accordingly, protrusions 203 assist in guiding the sheath mount 222 as it is displaced longitudinally.

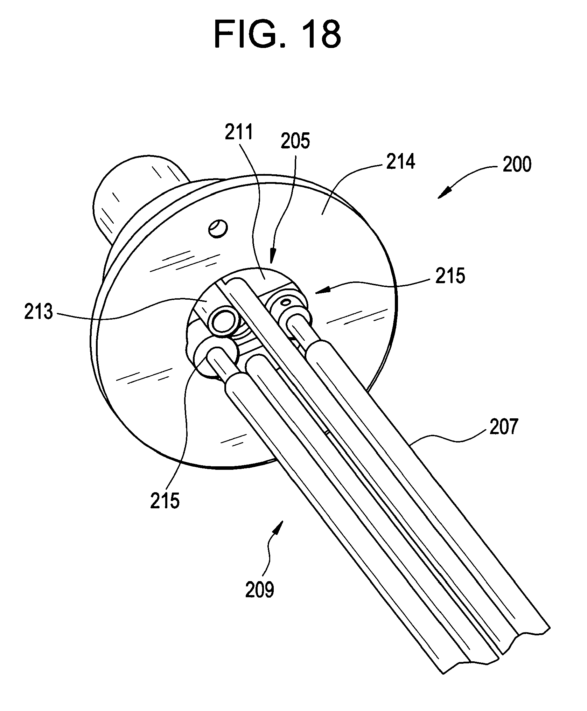

Fig. 18 is a proximal end of the handle system 200. Rotating potion 214 has a central opening 205 to receive a manifold (not shown) and guide rails 228, 230 with other central structure. Also received within opening 205 are two diametrically opposed drive screws 207, 209. Drive screws 207, 209 have spur gears 215 on their respective proximal ends. Spur gears 215 mesh with internal gear teeth 211 around the circumference of opening 205. As described above, drive screws 207, 209 rotate in the same direction as each other, and in the same direction as rotating portion 214 when turned.

Optionally, one of drive screws 207, 209 can be made to mesh with an idler gear 213. In this way, the drive screws 207, 209 are counter-rotating. Selecting the thread of the counter-rotating lead screw, in this case 209, to be an opposite direction of the other, 207, the sheath mount can be made to move longitudinally without applying any net torque. The torques applied to the sheath mount 222 by drive screws 207, 209 negate one another. This arrangement does present additional manufacturing steps, for example synchronization of the drive screws 207, 209 to avoid binding. It may, however, be considered worthwhile.

Drive screws 207, 209 and guide rails 228, 230 are arranged in an alternating manner around the longitudinal axis of the handle system 200, and of sheath mount 222. Drive screws are 207, 209 are received in sheath mount 22 at internally threaded holes (not shown).

The second embodiment may optionally include other optional features described with reference to the first embodiment. These include, without limitation, lock buttons 56, corresponding release buttons 100, strain relief 16, inner shaft seal 52, and/or variable pitch thread 74.

## Claims

1. A handle system of a delivery catheter for delivering a prosthetic implant device, the handle system comprising:
a first stationary portion;
at least one guide rail secured to the first stationary portion and extending generally longitudinally with the handle system;
a generally cylindrical second rotating portion rotatably connected to the first stationary portion, the second rotating portion having a threaded internal surface; and
a sheath mount secured to an outer sheath of the delivery catheter, including one or more bearing surfaces to engage with at least one of the one or more guide rails, and further having a generally cylindrical outer surface with a configuration to engage the internal threaded surface of the second rotating portion;
in which rotation of the second rotating portion longitudinally displaces the sheath mount by interaction of the threaded internal surface with the configuration of the generally cylindrical outer surface.

2. The handle system according to claim 1, in which the threaded internal surface comprises a helical male thread, and the configuration of the generally cylindrical outer surface comprises a helical channel.

3. The handle system according to claim 2, in which the helical male thread comprises a four-start helical male thread, and the helical thread channel comprises a four-start helical channel.

4. The handle system according to claim 1, in which the threaded internal surface comprises a thread channel, and the configuration of the generally cylindrical outer surface comprises one or more protrusions sized to engage the thread channel.

5. The handle system according to claim 4, in which the thread channel comprises a four-start thread channel, and the one or more protrusions comprises four protrusions.

6. The handle system according to claim 4, in which the thread channel varies in pitch along the longitudinal axis of the second rotating portion.

7. The handle system according to claim 6, in which the pitch of the thread channel at a proximal section of the second rotating portion is greater than the pitch of the thread channel at a distal section of the second rotating portion.

8. The handle system according to claim 1, in which at least one of the one or more guide rails comprises one or more ratchet teeth along its length, and the sheath mount further comprises a ratchet arm positioned to engage the ratchet teeth and operative to permit movement of the sheath mount in a first direction and to inhibit movement of the sheath mount in a second direction opposite the first direction.

9. The handle system according to claim 8, in which the at least one of the one or more guide rails further comprises a dead zone where no ratchet teeth are present to engage the ratchet arm or inhibit movement of the sheath mount in any direction.

10. The handle system according to claim 9, in which the dead zone is located at a distal end of the at least one of the one or more guide rails.

11. The handle system according to claim 1, in which the sheath mount further comprises at least one locking button captured in at least one radial recess, the locking button being biassed radially outward by a biassing means, and the second rotating portion comprises at least one through hole sized to admit at least a portion of the locking button.

12. The handle system according to claim 11, in which the biassing means comprises a compression spring.

13. The handle system according to claim 11, in which the recess comprises a first threaded post at the bottom thereof, and the locking button comprises a second threaded post on the underside thereof, and the biassing means comprises a helical spring engaging the first and second threaded posts, thereby capturing the locking button.

14. The handle system according to claim 11, in which the first stationary portion comprises a release button at a longitudinally aligned position with the through hole, and operative to depress the locking button radially inward of the through hole.

15. The handle system according to claim 14, in which the release button is captured to the first stationary portion.

16. The handle system according to claim 14, in which the release button further comprises a lock to retain the release button in the depressed position.

17. The handle system according to claim 11, in which the at least one through hole comprises a plurality of through holes.

18. The handle system according to claim 17, in which each of the plurality of through holes comprises a release button at a longitudinally aligned position with the through hole, and operative to depress the locking button radially inward of the through hole.

19. The handle system according to claim 1, further comprising a strain relief joined to the first stationary portion and at a distal tip of the handle system.

20. The handle system according to claim 19, in which the strain relief comprises one or more of polyethylene and polytetrafluoroethylene (PTFE).

21. The handle system according to claim 19, in which the strain relief comprises longitudinal ribs having longitudinal spaces between them, the longitudinal ribs defining a generally conical outer surface, and supporting an inner cylinder.

22. The handle system according to claim 21, in which the longitudinal spaces penetrate the inner cylinder.

23. The handle system according to claim 21, in which the longitudinal spaces extend to the distal tip of the strain relief.

24. The handle system according to claim 19, in which the strain relief comprises plural rows of openings extending a circumferential direction.

25. The handle system according to claim 24, in which the openings are staggered in longitudinal and circumferential directions.

26. The handle system according to claim 1, in which the first stationary portion comprises one or more circumferential ribs extending at least partially around the first stationary portion.

27. The handle system according to claim 1, in which the second rotating portion comprises one or more longitudinal ribs extending at least partially along the second rotating portion.

28. The handle system according to claim 1, further comprising a delivery catheter extending from the distal tip of the stationary portion, the delivery catheter having an inner core and an outer sheath which are longitudinally displaceable relative to one another.

29. The handle system according to claim 28, in which the delivery catheter is preloaded with one or more prosthetic implants at a distal tip thereof.

30. A handle system of a delivery catheter for delivering a prosthetic implant device, the handle system comprising:
a first stationary portion;
at least one guide rail secured to the first stationary portion and extending generally longitudinally with the handle system;
a second rotating portion rotatably connected to the first stationary portion, having a central opening with internal gear teeth;
one or more drive screws extending generally longitudinally with the handle system, each drive screw having a spur gear at a proximal end thereof, the spur gears communicating with the internal gear teeth of the central opening to rotate together with the second rotating portion; and
a sheath mount secured to an outer sheath of the delivery catheter, including one or more bearing surfaces to engage with at least one of the one or more guide rails, and further having one or more through holes with a configuration to engage one or more drive screws;
in which rotation of the second rotating portion longitudinally displaces the sheath mount by interaction of the drive screws with the configuration of the one or more through holes in the sheath mount.

31. The handle system according to claim 30, in which the one or more drive screws comprise a thread channel, and the configuration of the through holes comprises one or more inward protrusions sized to engage the thread channel.

32. The handle system according to claim 31, in which the thread channel varies in pitch along the longitudinal axis of the one or more drive screws.

33. The handle system according to claim 32, in which the pitch of the thread channel at a proximal section of the one or more drive screws is greater than the pitch of the thread channel at a distal section of one or more drive screws.

34. The handle system according to claim 30, in which at least one of the one or more guide rails comprises one or more ratchet teeth along its length, and the sheath mount further comprises a ratchet arm positioned to engage the ratchet teeth and operative to permit movement of the sheath mount in a first direction and to inhibit movement of the sheath mount in a second direction opposite the first direction.

35. The handle system according to claim 34, in which the at least one of the one or more guide rails further comprises a dead zone where no ratchet teeth are present to engage the ratchet arm or inhibit movement of the sheath mount in any direction.

36. The handle system according to claim 35, in which the dead zone is located at a distal end of the at least one of the one or more guide rails.

37. The handle system according to claim 30, in which the first stationary portion generally surrounds and encloses the at least one guide rail, the one or more drive screws, and the sheath mount.

38. The handle system according to claim 37, in which the sheath mount comprises one or more longitudinal recesses on an outer surface, and the first stationary portion comprises one or more longitudinal protrusions along at least a portion of its length, sized to be received in the one or more longitudinal recesses.

39. The handle system according to claim 37, in which the sheath mount further comprises at least one locking button captured in at least one radial recess, the locking button being biassed radially outward by a biassing means, and the first stationary portion comprises at least one through hole sized to admit at least a portion of the locking button.

40. The handle system according to claim 39, in which the biassing means comprises a compression spring.

41. The handle system according to claim 39, in which the recess comprises a first threaded post at the bottom thereof, and the locking button comprises a second threaded post on the underside thereof, and the biassing means comprises a helical spring engaging the first and second threaded posts, thereby capturing the locking button.

42. The handle system according to claim 39, in which the first stationary portion further comprises a release button at a longitudinally aligned position with the through hole, and operative to depress the locking button radially inward of the through hole.

43. The handle system according to claim 42, in which the release button is captured to the first stationary portion.

44. The handle system according to claim 43, in which the release button further comprises a lock to retain the release button in the depressed position.

45. The handle system according to claim 39, in which the at least one through hole comprises a plurality of through holes.

46. The handle system according to claim 45, in which each of the plurality of through holes comprises a release button at a longitudinally aligned position with the through hole, and operative to depress the locking button radially inward of the through hole.

47. The handle system according to claim 30, further comprising a strain relief joined to the first stationary portion and at a distal tip of the handle system.

48. The handle system according to claim 47, in which the strain relief comprises one or more of polyethylene and polytetrafluoroethylene (PTFE).

49. The handle system according to claim 47, in which the strain relief comprises longitudinal ribs having longitudinal spaces between them, the longitudinal ribs defining a generally conical outer surface, and supporting an inner cylinder.

50. The handle system according to claim 49, in which the longitudinal spaces penetrate the inner cylinder.

51. The handle system according to claim 49, in which the longitudinal spaces extend to the distal tip of the strain relief.

52. The handle system according to claim 47, in which the strain relief comprises plural rows of openings extending a circumferential direction.

53. The handle system according to claim 52, in which the openings are staggered in longitudinal and circumferential directions.

54. The handle system according to claim 30, in which the one or more drive screws comprises an even number of drive screws divided into a first half and a second half, the first half of the drive screws engaging the internal gear teeth via an idler gear to reverse the direction of rotation, and the first half of the drive screws threaded in the opposite direction from the second half.

55. The handle system according to claim 30, in which the first stationary portion comprises one or more circumferential ribs extending at least partially around the first stationary portion.

56. The handle system according to claim 30, in which the second rotating portion comprises one or more longitudinal ribs extending at least partially along the second rotating portion.

57. The handle system according to claim 30, further comprising a delivery catheter extending from the distal tip of the stationary portion, the delivery catheter having an inner core and an outer sheath which are longitudinally displaceable relative to one another.

58. The handle system according to claim 57, in which the delivery catheter is preloaded with one or more prosthetic implants at a distal tip thereof.
